(19)

Europäisches
Patentamt
European
Patent Office
Office européen
des brevets

(11) **EP 1 997 424 A1**

(12) **EUROPEAN PATENT APPLICATION**
published in accordance with Art. 158(3) EPC

(43) Date of publication:
**03.12.2008 Bulletin 2008/49**

(21) Application number: **07708301.2**

(22) Date of filing: **09.02.2007**

(51) Int Cl.:
*A61B 5/00* (2006.01)     *A61B 5/05* (2006.01)

(86) International application number:
**PCT/JP2007/052365**

(87) International publication number:
**WO 2007/108249 (27.09.2007 Gazette 2007/39)**

(84) Designated Contracting States:
**DE ES FR GB IT**

(30) Priority: **17.03.2006 JP 2006075249**

(71) Applicant: Omron Healthcare Co., Ltd.
**Kyoto 615-0084 (JP)**

(72) Inventors:
• **KINOSHITA, Yumi,**
**c/o Omron Healthcare Co., Ltd.**
**Kyoto-shi, Kyoto 6150084 (JP)**

• **TOGOE, Yasuyuki,**
**c/o Omron Healthcare Co., Ltd.**
**Kyoto-shi, Kyoto 6150084 (JP)**
• **SATO, Tetsuya,**
**c/o Omron Healthcare Co., Ltd.**
**Kyoto-shi, Kyoto 6150084 (JP)**

(74) Representative: **Wilhelms · Kilian & Partner**
**Patentanwälte**
**Eduard-Schmid-Strasse 2**
**81541 München (DE)**

(54) **BIOLOGICAL INFORMATION MEASUREMENT DEVICE**

(57)     An object of the invention is to provide a biological information measuring apparatus in which measurement result is displayed in various display modes. A biological information measuring apparatus 2 includes an input unit 16 which feeds display mode data used to determine a display mode of plural items of biological information, a storage unit in which the display mode data is stored, a measuring unit 6 which measures the plural items of biological information, a display unit 18 which determines the display mode of the biological information measured by the measuring unit 6, and a control unit 10 which causes the display unit 18 to display the measured biological information according to the display mode data stored in the storage unit.

Fig. 1

**Description**

TECHNICAL FIELD

The present invention relates to a biological information measuring apparatus.

BACKGROUND ART

Conventionally, there is known a living body measuring apparatus which sequentially switches to measure each region of a living body and displays measurement result in each region selected by a user (see Patent Document 1).
There is also known a body composition meter which compares past measurement data and latest measurement data and changes a display color according to the comparison result (see Patent Document 2).

Patent Document 1: Japanese Patent Laid-Open No. 2004-243148
Patent Document 2: Japanese Patent Laid-Open No. 2005-261488]

DISCLOSURE OF THE INVENTION

PROBLEMS TO BE SOLVED BY THE INVENTION

However, in the living body measuring apparatus disclosed in Patent Document 1, in order to display the measurement result, it is necessary that the user manually select a display item he or she desires to display, each time. In the body composition meter disclosed in Patent Document 2, when the measurement result is displayed, a display mode data is fixed although a change in measurement data can be known by the comparison with the past measurement data.
An object of the invention is to provide a biological information measuring apparatus in which the measurement result is displayed by various display modes.

MEANS FOR SOLVING THE PROBLEMS

In accordance with a first aspect of the invention, a biological information measuring apparatus includes a storage unit in which display mode data is stored, the display mode data being used to determine a display mode of plural items of biological information; a measuring unit which measures the plural items of biological information; a display unit which determines the display mode of the biological information measured by the measuring unit; and a control unit which produces the display mode data to control the display mode of the display unit according to the display mode data, the display mode data including data indicating display order of the plural items of biological information based on input data.
In accordance with a second aspect of the invention, a biological information measuring apparatus includes an input unit which feeds display mode data used to determine a display mode of plural items of biological information; a storage unit in which the display mode data is stored; a measuring unit which measures the plural items of biological information; a display unit which determines the display mode of the biological information measured by the measuring unit; and a control unit which causes the display unit to display the measured biological information according to the display mode stored in the storage unit.
In accordance with a third aspect of the invention, a biological information measuring apparatus includes a storage unit in which display mode data is stored, the display mode data being used to determine a display mode of plural items of biological information; a measuring unit which measures the plural items of biological information; a display unit which determines the display mode of the measured biological information; an input unit which provides an instruction for switching display of an item of the biological information displayed on the display unit; and a control unit which produces display-related data relating to the display of the biological information displayed on the display unit in response to the instruction from the input unit, stores display mode data based on the display-related data in the storage unit, and controls the display mode of the display unit according to the stored display mode data.
In accordance with a fourth aspect of the invention, a biological information measuring apparatus includes an input unit which feeds attribute data relating to a living body; a storage unit in which plural pieces of display mode data are prestored, the plural pieces of display mode data being used to determine a display mode of plural items of biological information; a measuring unit which measures the plural items of biological information; a display unit which determines the display mode of the biological information measured by the measuring unit; and a control unit which determines one piece of display mode data from plural pieces of display mode data based on the attribute data and causes the display unit to display the measured biological information according to the determined display mode data.

In accordance with a fifth aspect of the invention, a biological information measuring apparatus includes a storage unit in which plural items of target values are stored; a measuring unit which measures plural items of biological information; a display unit which determines a display mode of the biological information measured by the measuring unit; and a control unit which produces display mode data used to determine the display mode of the measured biological information according to result of comparison between the measured biological information and the target value for each item and causes display unit to display the biological information according to the display mode data.

EFFECT OF THE INVENTION

According to the invention, the measurement result can be displayed by various pieces of display mode data.

BRIEF DESCRIPTION OF THE DRAWINGS

Fig. 1 is a block diagram showing a circuit configuration of a biological information measuring apparatus according to an embodiment of the invention.
Fig. 2 is a schematic view showing the biological information measuring apparatus of the embodiment.
Fig. 3 is a block diagram showing a circuit configuration of a biological information measuring apparatus according to a modification of the embodiment.
Fig. 4 is a schematic view showing the biological information measuring apparatus of the modification of the embodiment.
Fig. 5 is a view showing a display unit and an input unit of an operation unit in a biological information measuring apparatus of Example 1.
Fig. 6 is a flowchart showing a procedure of setting display mode in the biological information measuring apparatus of Example 1.
Fig. 7 is a view showing a display unit and an input unit of an operation unit in a biological information measuring apparatus of a modification of Example 1.
Fig. 8 is a flowchart showing a procedure of setting display mode in the biological information measuring apparatus of the modification of Example 1.
Fig. 9 is a view showing a display unit and an input unit of an operation unit in a biological information measuring apparatus of Example 2.
Fig. 10 is a flowchart showing a procedure of determining display mode in the biological information measuring apparatus of Example 2.
Fig. 11 is a view showing a display time for each item in the display unit of the operation unit of Example 2.
Fig. 12 is a view showing a display unit and an input unit of an operation unit in a biological information measuring apparatus of a modification of Example 2.
Fig. 13 is a flowchart showing a procedure of determining display mode in the biological information measuring apparatus of the modification of Example 2.
Fig. 14 is a view showing a display unit and an input unit of an operation unit in a biological information measuring apparatus of Example 3.
Fig. 15 is a flowchart showing a procedure of determining display mode data in the biological information measuring apparatus of Example 3.
Fig. 16 is a view showing a display unit and an input unit of an operation unit in a biological information measuring apparatus of Example 4.
Fig. 17 is a flowchart showing a procedure of setting display mode in the biological information measuring apparatus of Example 4.

EXPLANATIONS OF LETTERS OF NUMERALS

2      biological information measuring apparatus
4      operation unit
6      measuring unit
8      cable
10     control unit
12     memory

14     power supply
16     input unit
18     display unit
20     sensor
22     computation unit
24     interface
26     switch button
28     determination button
30     display order changing mode button
32     item-A button
34     item-B button
36     item-C button
38     reset button
40     ten key
42     setting button

BEST MODE FOR CARRYING OUT THE INVENTION

[Embodiment]

Exemplary embodiment of the invention will be described in detail with reference to the drawings. However, a technical scope of the invention is not limited to a size, a material, a shape, and a relative layout of a component described in the embodiment unless otherwise stated.

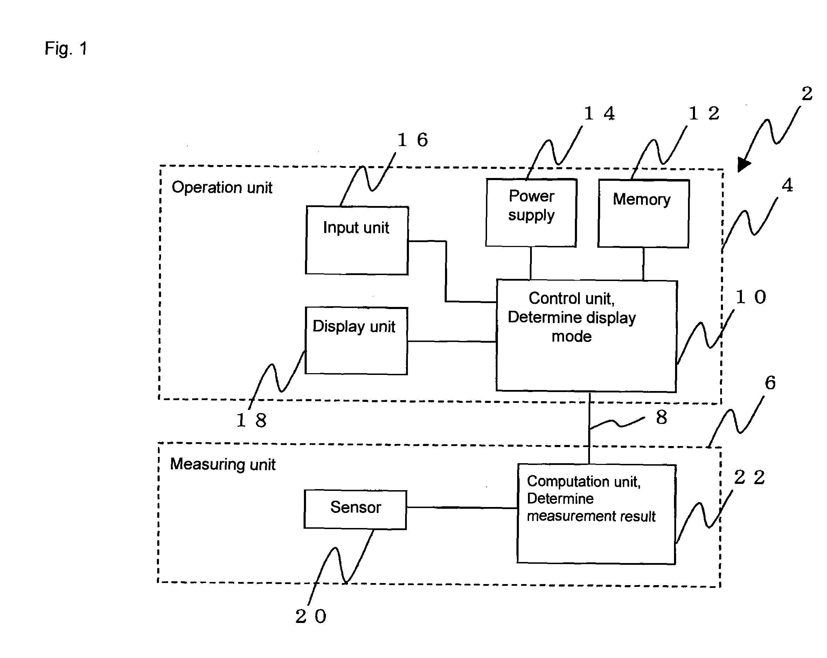

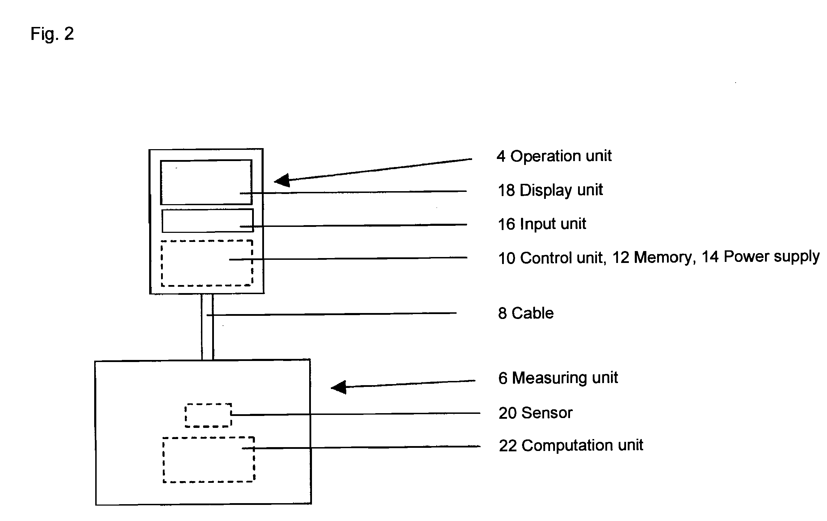

A biological information measuring apparatus according to an embodiment of the invention will be described with reference to Figs. 1 and 2. Fig. 1 is a block diagram showing a circuit configuration of a biological information measuring apparatus according to an embodiment of the invention. Fig. 2 is a schematic view showing the biological information measuring apparatus of the embodiment.

As shown in Fig. 1, a biological information measuring apparatus 2 of the embodiment includes an operation unit 4 and a measuring unit 6. The operation unit 4 and the measuring unit 6 are connected through a cable 8.

As shown in Fig. 2, in the operation unit 4 of the biological information measuring apparatus 2, a control unit 10, a memory 12, and a power supply 14 are accommodated in a chassis, and an input unit 16 and a display unit 18 are formed so as to be visible from the outside. The measuring unit 6 is formed such that a sensor 20 and a computation unit 22 are accommodated in a chassis. For the sensor 20, depending on a kind of the biological information measuring apparatus 2, an electrode and the like connected to the sensor 20 may directly contact a living body. Depending on a kind of the biological information measuring apparatus 2, the operation unit 4 and the measuring unit 6 may be accommodated in the same chassis.

(1. Operation Unit)

The operation unit 4 includes the input unit 16 which is used for inputs of various pieces of information, the display unit 18 which displays the information fed from the input unit 16 and result measured by the measuring unit 6, the memory 12 in which the information to be displayed on the display unit 18 is stored, the control unit 10 which controls operations of the input unit 16, display unit 18, and memory 12, and the power supply 14. A physically-pressed button and a touch panel can be used as the input unit 16. In the case where the touch panel is used, the input unit 16 and the display unit 18 may integrally be provided.

(1.1 Memory)

Display mode data used to determine a display mode of plural items of biological information is stored in the memory 12 (corresponding to "storage unit" of the invention). The display mode data is data relating to a size or a color of characters and the data which determines display order of the plural items of biological information and display mode of the plural items of biological information. The color data is data relating to a color of characters and graphs or background color of characters. The plural items of biological information are described later.

(1.2 Display Unit)

The display unit 18 displays the biological information measured by the measuring unit 6. The display unit 18 displays the input information and the like fed from or selected by (1) the input unit 16 in addition to the measured biological information. The "biological information measured by the measuring unit 6" shall mean data in which the measured biological information (comparison result, time-series graph, point memory (result in comparison with data at a certain point), and determination result (for example, degree of obesity and achievement level of target value)). A display device such as a color liquid crystal display is used as the display unit 18.

(1.3 Control Unit)

The control unit 10 is a Central Processing Unit (CPU). The control unit 10 produces data indicating the display order of the plural items of biological information based on the fed data, and the display mode of the display unit 18 is controlled according to the display mode data. Data fed from the input unit 16 or computation unit 22 can be cited as an example of the data fed into the control unit 10. The control unit 10 may store the display mode data in the memory 12 and control the display mode of the display unit 18 according to the stored display mode data.

(Data fed from Input Unit 16)

Examples of the data fed from the input unit 16 include data used to determine the display order of the biological information fed by the user and data indicating an instruction of display switch of display items. The control unit 10 produces the display mode data based on the data fed from the input unit 16.

(Data fed from Computation Unit 23)

The data fed from the computation unit 22 (CPU) is the measurement data of the biological information measured by the measuring unit 6. The control unit 10 produces the display mode data by comparing the measurement data of the biological information measured by the measuring unit 6 to comparison object data prestored in the memory 12 (for example, a target value such as a statistical average value such as a personal attribute (age and sex) identical to the user or past measurement data).

(2. Measuring Unit)

The measuring unit 6 measures the biological information relating to plural items (kinds). The measuring unit 6 includes the sensor 20 and the computation unit 22. The sensor 20 detects data relating to the living body. The computation unit 22 performs various computations in order to obtain the plural items of biological information from the data obtained by the sensor 20. The computation result (measurement data) is transferred through the cable 8 to the control unit 10 on the side of the operation unit 4. The biological information directly measured with the sensor 20, the measurement data, and the data in which the measurement data is processed are displayed on the display unit 18.

(Modification)

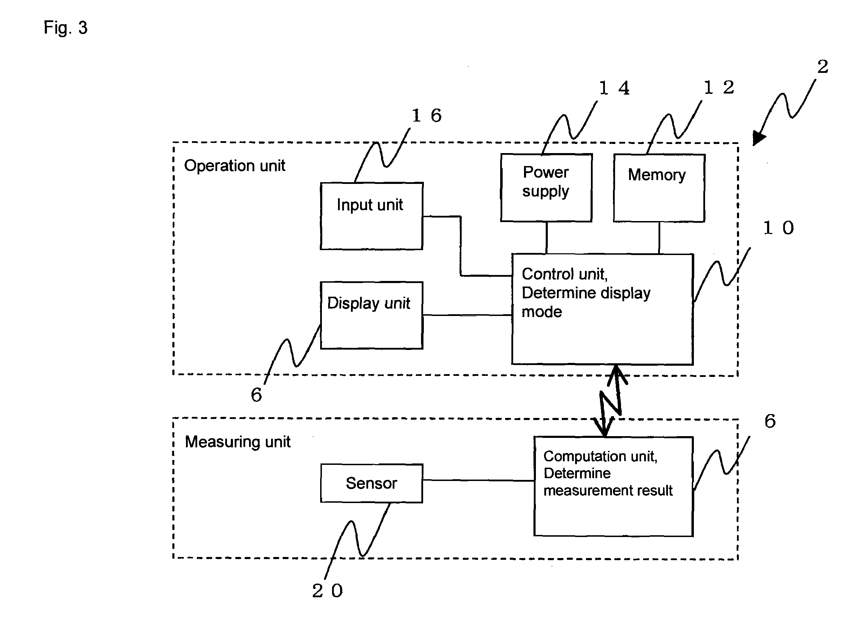

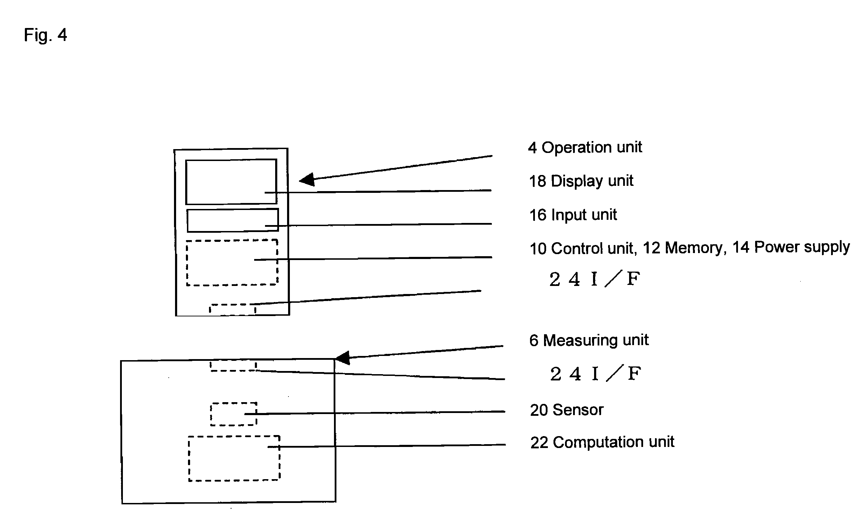

Fig. 3 is a block diagram showing a circuit configuration of a biological information measuring apparatus 2 according to a modification of the embodiment. Fig. 4 is a schematic view showing the biological information measuring apparatus 2 of the modification of the embodiment. In the embodiment, the operation unit 4 and the measuring unit 6 are connected through the cable 8. Alternatively, a wireless communication interface 24 may be provided. A communication technique such as infrared communication and Bluetooth can be used as the communication method.

[Examples]

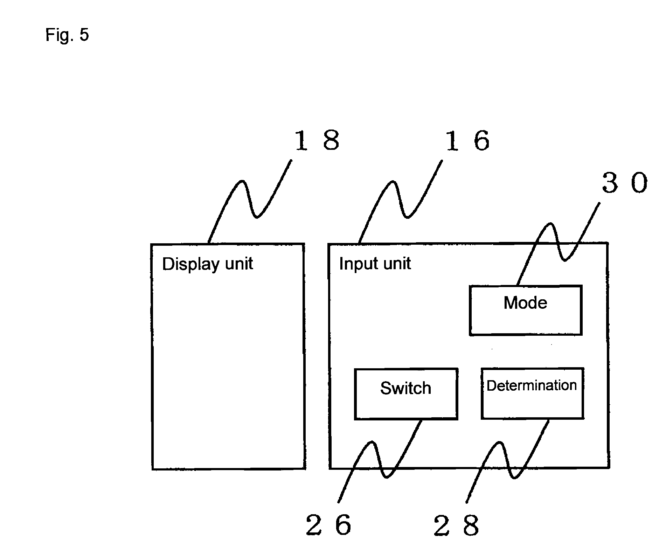

Examples of the invention will be described below. In the following Examples, the biological information measured by the body composition meter (biological information measuring apparatus) is described by way of example. Examples will be described with reference to the configuration described in the above embodiment and Figs. 5 to 17. The same component as the embodiment is designated by the same numeral.

(Example 1)

In Example 1, a processing procedure of setting the display mode of the plural items of biological information will be described in order to display the measurement result on the display unit 18 in the display mode according to a preference of the user.

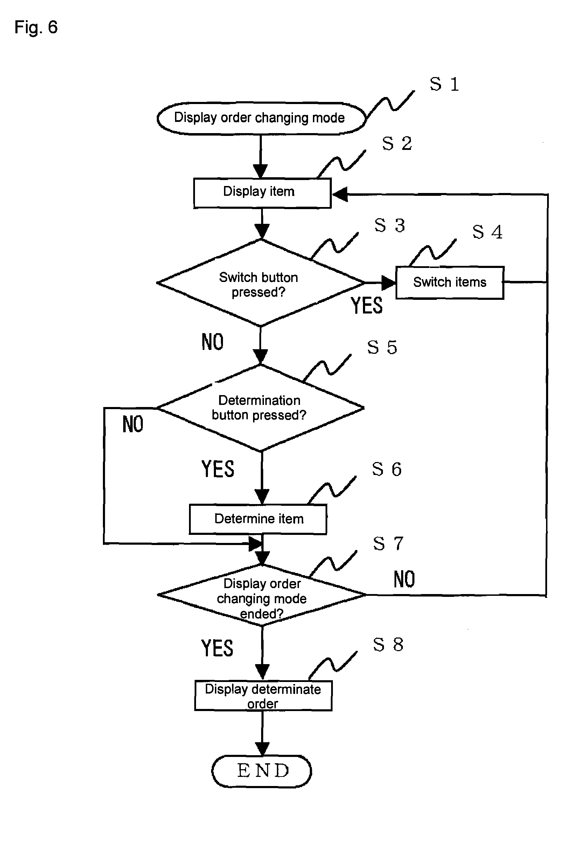

Fig. 5 is a view showing the display unit 18 and input unit 16 of the operation unit 4 in the biological information measuring apparatus 2 of Example 1. Fig. 6 is a flowchart showing a procedure of setting display mode data in the biological information measuring apparatus 2 of Example 1.

In Fig. 5, the input unit 16 is used to feed the display mode data which is used to determine the display mode of the plural items of biological information. A switch button 26, a determination button 28, a display order changing mode button 30 are provided in the input unit 16.

Assuming that the plural items of biological information include "weight value", "body fat percentage", and "basal metabolism", the description will be made below. The display mode data shall mean data indicating the display order of "A: weight value", "B: body fat percentage", and "C: basal metabolism".

The display mode data is stored in the memory 12 such that "A: weight value", "B: body fat percentage", and "C: basal metabolism" are sequentially displayed as an initial setting. For example, the initial setting data of "1,A,B,C" is stored in the memory 12 in the form of the table. "1" is data indicating the number of display items, and the control unit 10 causes the display unit 18 to display the measurement result item by item. The initial setting data is data indicating that A, B, and C are displayed one by one every time the switch button 26 is pressed. When the user presses the switch button 26 while "basal metabolism" is displayed, the control unit 10 causes the display unit 18 to display "weight value". The display unit 18 may simultaneously display "A: weight value", "B: body fat percentage", and "C: basal metabolism". At this point, the control unit 10 may change the color or size of the characters or background and display the selected item such that the item is highlighted. In the case where the two items are simultaneously displayed, for example, the initial setting data becomes "2,A,B,C".

Assuming that the initial setting data is "1,A,B,C", the method of setting the display order will be described below.

The user turns on the power supply 14 of the biological information measuring apparatus, and the user presses the display order changing mode button 30 to set the biological information measuring apparatus at "display order changing mode" (S1). Then, the control unit 10 causes the display unit 18 to display "weight value" according to the initial setting data read from the memory 12 (S2).

When the user presses the switch button 26 once (S3), the control unit 10 causes the display unit 18 to display "body fat percentage" (S4). At this point, when the user presses the determination button 28 (S5), the control unit 10 sets "B: body fat percentage" at a priority of 1 to write "B: body fat percentage" in the table of the memory 12 (S6).

When the user presses the switch button 26 twice (S3), the control unit 10 causes the display unit 18 to display "weight value" (S4). At this point, when the user presses the determination button 28 (S5), the control unit 10 sets "A: weight value" at a priority of 2 to write "A: weight value" in the table of the memory 12 (S6).

When the user presses the switch button 26 twice (S3), the control unit 10 causes the display unit 18 to display "basal metabolism" (S4). At this point, when the user presses the determination button 28 (S5), the control unit 10 sets "C: basal metabolism" at a priority of 3 to write "C: basal metabolism" in the table of the memory 12 (S6).

When the user presses the display order changing mode button 30 (S7), the control unit 10 ends the display order changing mode, and the control unit 10 cause the display unit 18 to display the determinate display order (S8).

"1,B,A,C" which is of the display mode data is stored in the memory 12. The display mode data of "1,B,A,C" indicates that "body fat percentage", "weight value", and "basal metabolism" are sequentially displayed item by item.

When the measuring unit 6 measures the plural items of biological information, the control unit 10 receives the biological information measured by the measuring unit 6, the control unit 10 reads the display mode data stored in the memory 12, and the control unit 10 causes the display unit 18 to display the measured biological information in the order of the "body fat percentage", "weight value", and "basal metabolism" according to the display mode data.

In Example 1, the measurement result can be displayed on the display unit 18 in the display mode according to the preference of the user.

The switch button 26 and the determination button 28 are provided, so that the operation unit 4 can be formed by fewer buttons.

(Modification)

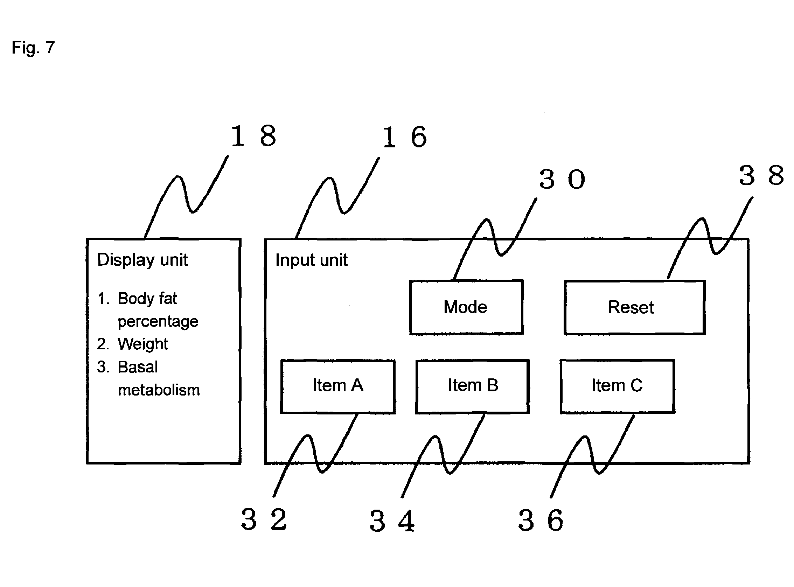

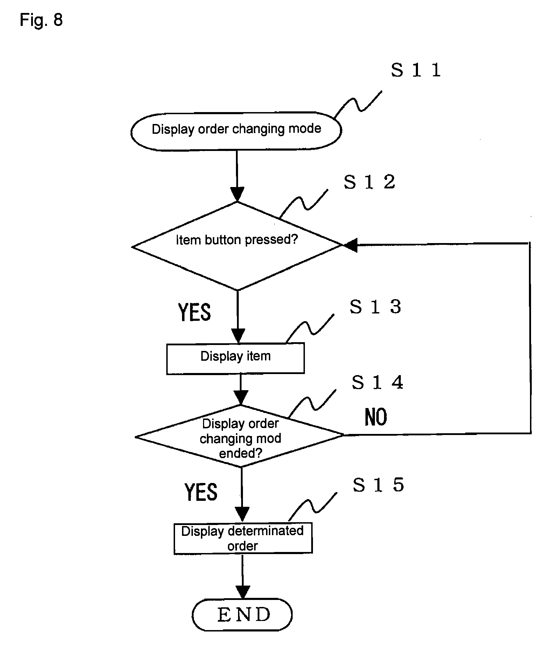

An example in which the user sets the display mode data will be described below with reference to Figs. 7 and 8. Fig. 7 is a view showing the display unit 18 and input unit 16 of the operation unit 4 in the biological information measuring apparatus 2 of a modification of Example 1. Fig. 8 is a flowchart showing a procedure of setting display mode data in the biological information measuring apparatus 2 of the modification of Example 1.

In Fig. 7, an item-A button 32, an item-B button 34, an item-C button 36, a reset button 38, and the display order changing mode button 30 are provided in the input unit 16. The item-A button 32 is a button which selects "A: weight value". The item-B button 34 is a button which selects "B: body fat percentage", and the item-C button 36 is a button which selects "C: basal metabolism".

A method of setting the display order will be described below as an example in which the initial setting data is not utilized.

The user turns on the power supply 14 of the biological information measuring apparatus, and the user presses the display order changing mode button 30 to set the biological information measuring apparatus at "display order changing mode" (S11). At this point, the control unit 10 causes the display unit 18 to display "Press button in the order in which you want the items to be displayed".

When the user presses the item-B button 34 (S12), the control unit 10 causes the display unit 18 to display "1. body fat percentage" (S13). Then, the control unit 10 sets "B: body fat percentage" at the priority of 1 to write "B: body fat percentage" in the table of the memory 12.

When the user presses the item-A button 32 (S12), the control unit 10 causes the display unit 18 to display "2. weight" (S13). Then, the control unit 10 sets "A: weight value" at the priority of 2 to write "A: weight value" in the table of the memory 12.

When the user presses the item-C button 36 (S12), the control unit 10 causes the display unit 18 to display "3. basal metabolism" (S13). Then, the control unit 10 sets "C: basal metabolism" at the priority of 2 to write "C: basal metabolism" in the table of the memory 12. When the reset button 38 is pressed, the display of the display unit 18 returns to the initial state (no display), and the data stored in the memory 12 is erased.

When the user presses the display order changing mode button 30 (S14), the control unit 10 ends the display order changing mode, and the control unit 10 causes the display unit 18 to display the determinate display order.

"1,B,A,C" which is of the display mode data is stored in the memory 12. The display mode data of "1,B,A,C" indicates that "body fat percentage", "weight value", and "basal metabolism" are sequentially displayed item by item.

When the measuring unit 6 has measured the plural items of biological information, the control unit 10 receives the biological information measured by the measuring unit 6, the control unit 10 reads the display mode data stored in the memory 12, and the control unit 10 causes the display unit 18 to display the biological information in the order of the "body fat percentage", "weight value", and "basal metabolism" according to the display mode data.

Therefore, the measurement result can be displayed on the display unit 18 in the display mode according to the preference of the user.

The input unit 16 includes the plural item buttons relating to the biological information which should be displayed on the display unit 18, so that the user can easily select the display mode data.

(Example 2)

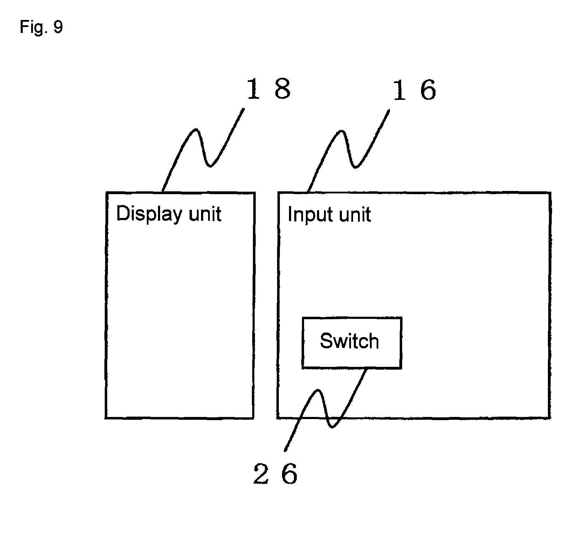

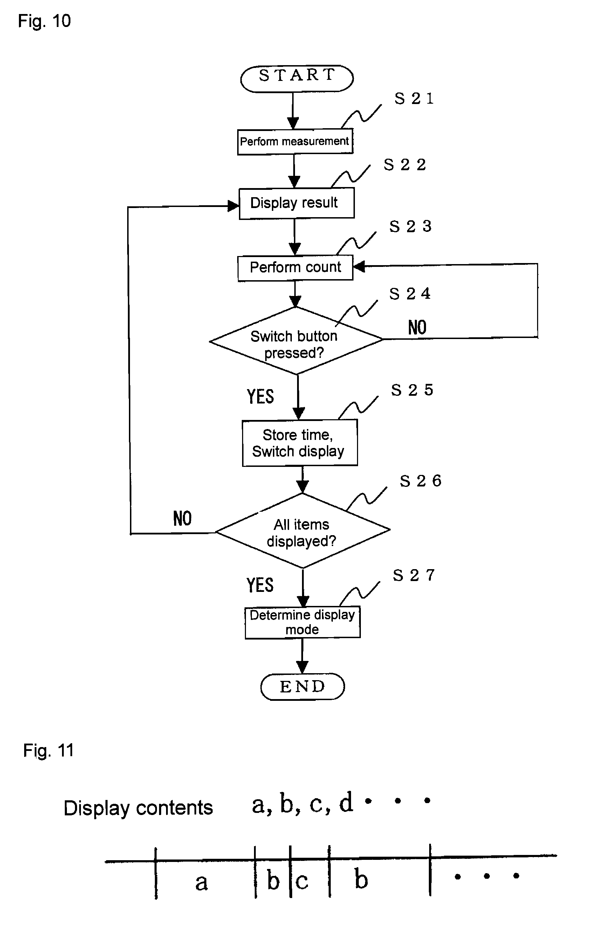

In Example 2, a processing procedure of determining the display mode of the plural items of biological information will be described in order to display the measurement result on the display unit 18 in the display mode according to a usage characteristic (display time) of the user. Fig. 9 is a view showing the display unit 18 and input unit 16 of the operation unit 4 in the biological information measuring apparatus 2 of Example 2. Fig. 10 is a flowchart showing a procedure of determining display mode data in the biological information measuring apparatus 2 of Example 2. Fig. 11 is a view showing the display time for each item in the display unit 18 of the operation unit 4 of Example 2.

In Fig. 9, the switch button 26 is provided in the input unit 16 in order to provide an instruction for switching the display of the item of the biological information displayed on the display unit 18. The display unit 18 determines the display mode of the plural items of biological information measured by the measuring unit 6.

At this point, the plural items of biological information include "weight value", "body fat percentage", and "basal metabolism". The display mode data is data which indicates the display order of "A: weight value", "B: body fat percentage", and "C: basal metabolism".

The display mode data used to determine the display mode of the plural items of biological information is prestored in the memory 12. The display mode data is stored in the memory 12 such that "A: weight value", "B: body fat percentage", and "C: basal metabolism" are sequentially displayed as the initial setting. For example, the initial setting data of "1,A,B,C" is stored in the memory 12 in the form of the table. The initial setting data is data which indicates that "weight value", "body fat percentage", and "basal metabolism" are sequentially displayed one by one every time the switch button 26 is pressed. When the user presses the switch button 26 while "basal metabolism" is displayed, the control unit 10 causes the display unit 18 to display "weight value".

After the user turns on the power supply 14 of the biological information measuring apparatus, the measuring unit 6 measures the biological information (weight value, body fat percentage, and basal metabolism) (S21), the control unit 10 causes the display unit 18 to display "weight value" measured by the measuring unit 6 according to the initial setting data read from the memory 12 (S22). While the control unit 10 causes the display unit 18 to display "weight value", the control unit 10 starts to count a display time (corresponding to "display-related data" of the invention) (S23).

When the user presses the switch button 26 once (S24), the control unit 10 causes the display unit 18 to display "body fat percentage". The control unit 10 ends the count of the display time, and the control unit 10 stores the time during which "weight value" is displayed in the memory 12 (S25). At this point, it is assumed that the time during which "weight value" is displayed is 30 seconds. The control unit 10 starts to count a display time during which "body fat percentage" is displayed on the display unit 18 (S23).

When the user presses the switch button 26 once (S24), the control unit 10 causes the display unit 18 to display "basal metabolism". The control unit 10 ends the count of the display time, and the control unit 10 stores the time during which "body fat percentage" is displayed in the memory 12 (S25). At this point, it is assumed that the time during which "body fat percentage" is displayed is one minute. The control unit 10 starts to count a display time during which "basal metabolism" is displayed on the display unit 18 (S23).

When the user presses the switch button 26 once (S24), the control unit 10 causes the display unit 18 to display "weight value". The control unit 10 ends the count of the display time, and the control unit 10 stores the time during which "basal metabolism" is displayed in the memory 12 (S25). At this point, it is assumed that the time during which "basal metabolism" is displayed is 45 seconds.

In the case where all the items are displayed, the control unit 10 produces the display mode data from the display time of each item, and the control unit 10 stores the display mode data in the memory 12 in the form of the table (S27). The control unit 10 determines the priority of the item to be displayed in the descending order of the display time from the display times (weight value for 30 seconds; body fat percentage for one minute; and basal metabolism for 45 seconds) stored in the memory 12. That is, the control unit 10 determines the display order of "body fat percentage", "basal metabolism", and "weight value". For example, the control unit 10 produces the display mode data of "1,B,C,A" to store the display mode data of "1,B,C,A" in the memory 12. The control unit 10 controls the display mode data of the display unit 18 from the next measurement according to the stored display mode data.

In Example 2, the measurement result can be displayed on the display unit 18 in the display mode based on the usage characteristic (display time) of the user.

In Example 2, the display mode data is produced when all the items are displayed. Alternatively, the same item may be displayed plural times. In such cases, as shown in Fig. 11, the control unit 10 stores the display time of the item on the display unit 18 in the memory 12. For example, the control unit 10 may add the display time for each item when a predetermined time has elapsed since the initial display is started and produce the display mode data based on the addition result. According to this method, the usage characteristic can more correctly be reflected on the display mode.

In Example 2, the count of the display time is started from the moment it was displayed. Alternatively, the count of the display time may be started after a predetermined time has elapsed since the start of the display. Therefore, in the case of the many items, the display time of each item is not counted when the switch button 26 is continuously pressed plural times, so that the usage characteristic of the user can properly be reflected on the display mode data.

(Modification)

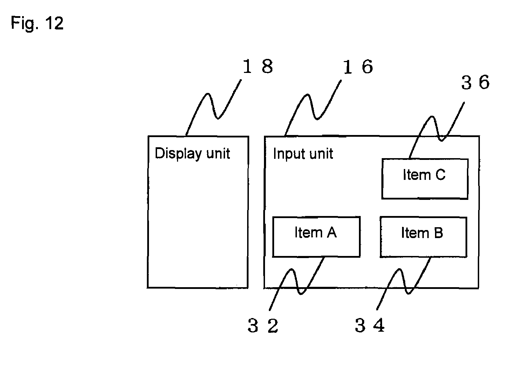

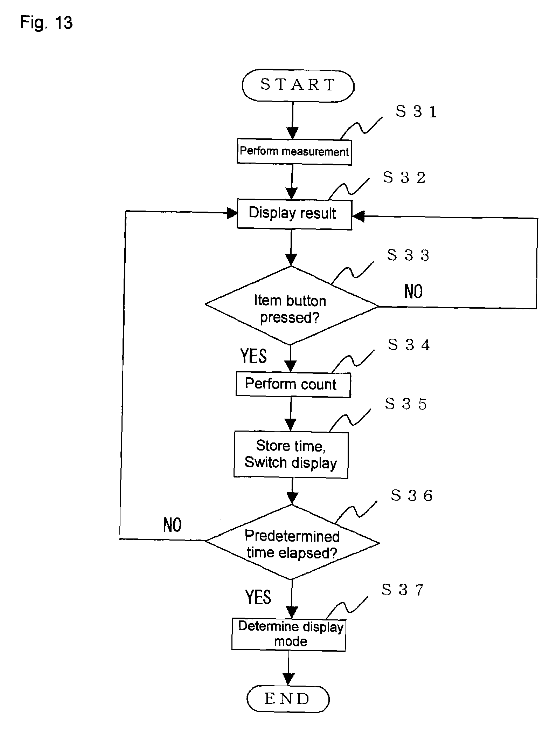

A processing procedure of determining the display mode data of the plural items of biological information will be described in order to display the measurement result on the display unit 18 in the display mode based on the usage characteristic (the number of display times) of the user. An example in which the display mode data is determined will be described below with reference to Figs. 12 and 13. Fig. 12 is a view showing the display unit 18 and input unit 16 of the operation unit 4 in the biological information measuring apparatus 2 of a modification of Example 2. Fig. 13 is a flowchart showing a procedure of determining display mode data in the biological information measuring apparatus 2 of the modification of Example 2.

In Fig. 12, the item-A button 32, the item-B button 34, the item-C button 36 are provided in the input unit 16 in order to provide the instruction for switching the display of the biological information item displayed on the display unit 18. The item-A button 32 is a button which selects "A: weight value". The item-B button 34 is a button which selects "B: body fat percentage", and the item-C button 36 is a button which selects "C: basal metabolism". The display unit 18 determines the display mode of the plural items of biological information measured by the measuring unit 6.

The display mode data used to determine the display mode of the plural items of biological information is prestored in the memory 12. The display mode data is stored in the memory 12 such that "A: weight value", "B: body fat percentage", and "C: basal metabolism" are sequentially displayed as the initial setting. For example, the initial setting data of "1,A,B,C" is stored in the memory 12 in the form of the table. When the measurement result of the measuring unit 6 is transferred to the control unit 10, the control unit 10 controls the display mode such that the displayed items are automatically switched at predetermined time intervals. When the predetermined time has elapsed since "basal metabolism" is displayed, the control unit 10 causes the display unit 18 to display "weight value".

After the user turns on the power supply 14 of the biological information measuring apparatus, when the measuring unit 6 has measured the biological information (weight value, body fat percentage, and basal metabolism) (S31), the control unit 10 causes the display unit 18 to switch to display "weight value", "body fat percentage", and "basal metabolism" measured by the measuring unit 6 at predetermined time intervals according to the initial setting data read from the memory 12 (S32).

(1) When the user presses the item-B button 34 (S33), the control unit 10 adds "1" to the number of display times of the item-B button 34 to write the added number of display times in the table of the memory 12, and the control unit 10 causes the display unit 18 to display "body fat percentage" (S34 and S35). The initial value of the number of display times of each item is set at "0".

(2) Then, when the user presses the item-A button 32 (S33), the control unit 10 adds "1" to the number of display times of the item-A button 32 to write the added number of display times in the table of the memory 12, and the control unit 10 causes the display unit 18 to display "weight value" (S34 and S35).

(3) Then, the user presses the item-B button 34 (S33), the control unit 10 adds "1" to the number of display times of the item-B button 34 to write the added number of display times in the table of the memory 12, and the control unit 10 causes the display unit 18 to display "body fat percentage" (S34 and S35).

(4) Then, when the user presses the item-C button 36 (S33), the control unit 10 adds "1" to the number of display times of the item-C button 36 to write the added number of display times in the table of the memory 12, and the control unit 10 causes the display unit 18 to display "basal metabolism" (S34 and S35). At this point, when the user further continuously presses the item-C button 36 four times, control unit 10 adds "4" to the number of display times of the item-C button 36 to write the added number of display times in the table of the memory 12, and the control unit 10 causes the display unit 18 to display "basal metabolism" (S34 and S35).

After a predetermined time has elapsed since the initial display, the control unit 10 produces the display mode data from the display time of each item, and the control unit 10 stores the display mode data in the memory 12in the form of the table (S36 and S37). At this point, the control unit 10 determines the priority of the displayed item in the descending order of the number of display times from the numbers of display times (weight value of one time; body fat percentage of twice; and basal metabolism of five times) stored in the memory 12. That is, the control unit 10 determines the display order of "basal metabolism", "body fat percentage", and "weight value". For example, the control unit 10 produces the display mode data of "1,B,C,A" to store the display mode data of "1,B,C,A" in the memory 12. The control unit 10 controls the display mode data of the display unit 18 in the order of "basal metabolism", "body fat percentage", and "weight value" from the next measurement according to the stored display mode data.

Therefore, the measurement result can be displayed on the display unit 18 in the display mode based on the usage characteristic (the number of display times) of the user.

(Example 3)

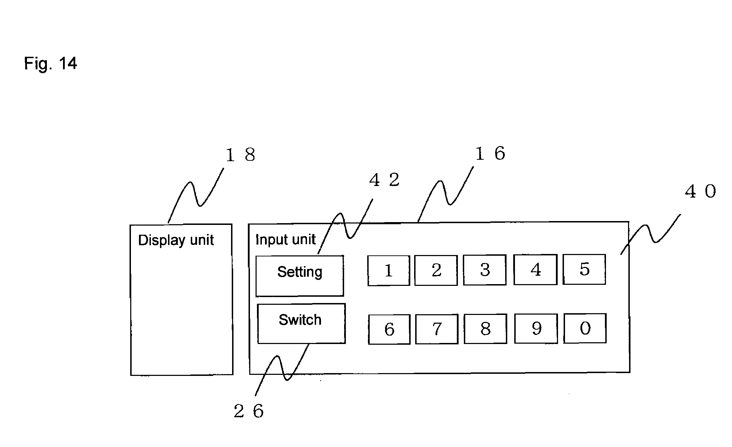

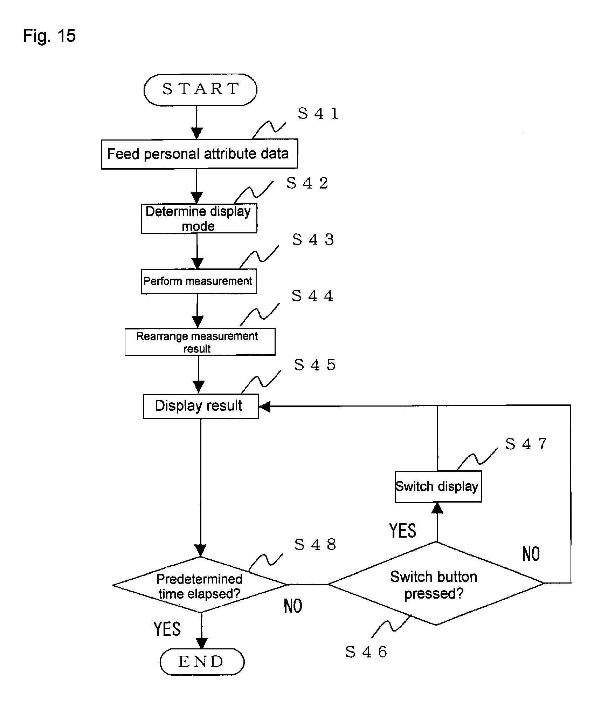

In Example 3, a processing procedure of determining the display mode data of the plural items of biological information will be described in order to display the measurement result on the display unit 18 in the display mode based on the personal attribute (such as age, sex, and body height) of the user. Fig. 14 is a view showing the display unit 18 and input unit 16 of the operation unit 4 in the biological information measuring apparatus 2 of Example 3. Fig. 15 is a flowchart showing a procedure of determining display mode data in the biological information measuring apparatus 2 of Example 3.

In Fig. 14, the switch button 26 which provides the instruction for switching the display item of the biological information displayed on the display unit 18 and a ten key 40 and a setting button 42 which are used to feed attribute data relating to the living body such as the body height, age, and sex of the user are provided in the input unit 16. The switch button 26 is used to switch the items to be displayed after the measurement or to feed the attribute data. The display unit 18 displays the plural items of biological information measured by the measuring unit 6 or the attribute data fed by the ten key 40.

In Example 3, plural pieces of display mode data used to determine the display mode of the plural items of biological information are prestored in the memory 12 in the form of the table.

For example, it is assumed that three pieces of display mode data are stored in Table 1 of the memory 12 while correlated with the age and sex.

(Table 1)

| 1. | 20,1,1,5,4 |
| 2. | 30,2,4,2,1 |
| 3. | 40,2,6,3,1 |

(Table 2)

| 1. | female |
| 2. | male |

(Table 3)

| 1. | weight value |

(continued)

2.     body fat percentage
3.     basal metabolism
4.     skeleton muscle ratio
5.     subcutaneous fat
6.     physical age

Table 1 is a table of the display mode data used to determine the display mode of the plural items of biological information. Table 2 is a table indicating the sex, and Table 3 is a table indicating the plural items of biological information. At this point other items of the biological information include a visceral fat level, BMI, and the like. For the body fat percentage, a total body, a body trunk, a right arm, a left arm, a right leg, and a left leg may be set at measurement and display targets. For the skeleton muscle ratio, the total body, the body trunk, the right arm, the left arm, the right leg, and the left leg may be set at the measurement and display targets.

Table 1 is a table which indicates a display mode data number, the age, the sex, and the display order from the left. The display mode data on the first line of Table 1 is data indicating that a weight value (1), a subcutaneous fat ratio (5), and a skeleton muscle ratio (4) are sequentially displayed for a female (1) in her twenties.

The following description will be made with reference to Fig. 15. The user operates the ten key 40 of the input unit 16 to feed the personal attribute data (S41). When the user presses the setting button 42 of the input unit 16, the control unit 10 encourages the user to feed the attribute data in the order of the age and the sex through the display unit 18. For example, the user feeds "25" with the ten key 40 when a message for encouraging the user to feed the "age" is displayed on the display unit 18. Then, when the user presses the switch button 26, the control unit 10 stores the age data of "25" in the memory 12, and the control unit 10 causes the display unit 18 to display a message for encouraging the user to feed "sex". The user feeds the sex data of "1" indicating the female using the ten key 40. The control unit 10 determines the display mode data (first line) from the plural pieces of display mode data (pieces of data of first to third lines) of Table 1 based on the fed attribute data (25,1) (S42). The control unit 10 causes the display unit 18 to display the measurement result in the order of the weight value (1), subcutaneous fat ratio (5), and the skeleton muscle ratio (4) based on the fed attribute data. It is not always necessary to feed the attribute data every time the attribute data is measured, and the control unit 10 may store the determinate display mode data in the memory 12 as the data applied to the measurement result.

Then, the user performs the measurement with the biological information measuring apparatus 2. The measuring unit 6 detects the plural items of biological information using the sensor 20, and the computation unit 22 produces the measurement data relating to each item (S43). The computation unit 22 transfers the measurement data to the control unit 10. The control unit 10 rearranges the received pieces of measurement data according to the determinate display mode data, and the control unit 10 causes the display unit 18 to display "weight value" (S44 and S45). When the control unit 10 detects that the switch button 26 is pressed (S46), the control unit 10 causes the display unit 18 to display "subcutaneous fat ratio" (S47 and S45). Then, the control unit 10 detects that the switch button 26 is pressed (S46), the control unit 10 causes the display unit 18 to display "skeleton muscle ratio" (S47 and S45). The control unit 10 turns off the power supply 14 when a predetermined time has elapsed since the display start (S48). The display mode of the measurement result is not limited to the method of displaying the measurement result one by one, but the method of displaying at least two measurement results may be listed.

Thus, in Example 3, the measurement result can be displayed on the display unit 18 in the display mode based on the personal attribute (such as age, sex, and body height) of the user.

(Example 4)

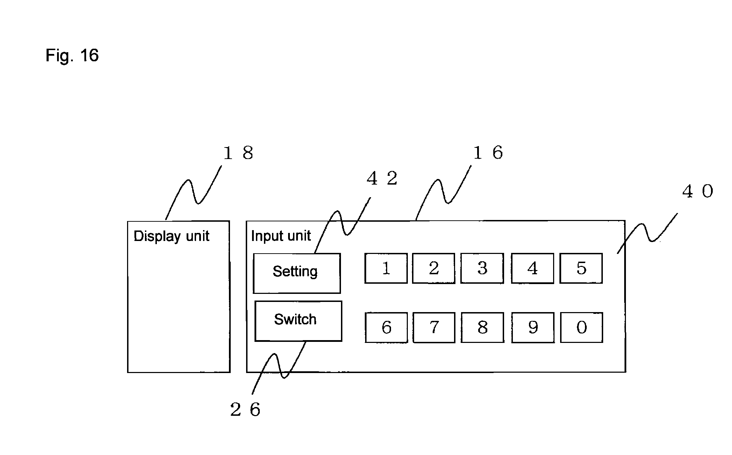

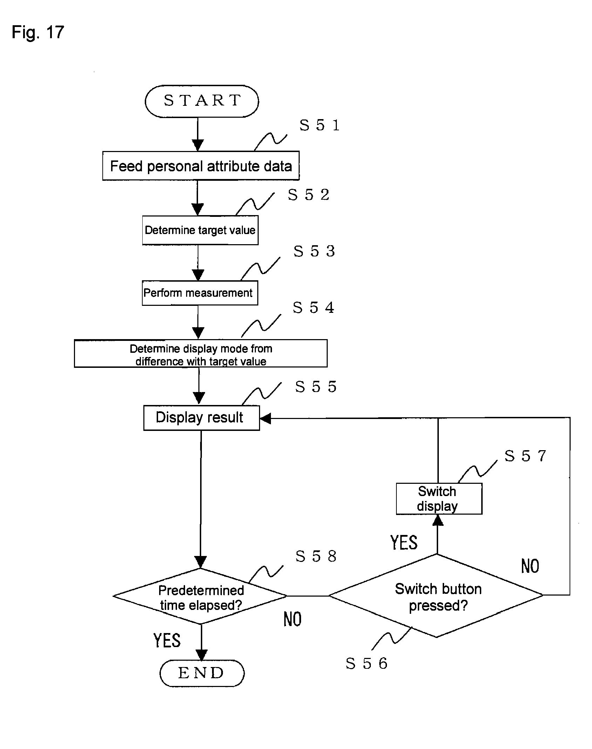

In Example 4, a processing procedure of determining the display mode data of the plural items of biological information will be described in order to display the measurement result on the display unit 18 in the display mode based on measurement result comparison of the biological information on the user. Fig. 16 is a view showing the display unit 18 and input unit 16 of the operation unit 4 in the biological information measuring apparatus 2 of Example 4. Fig. 17 is a flowchart showing a procedure of setting display mode data in the biological information measuring apparatus 2 of Example 4.

In Fig. 16, the switch button 26 which provides the instruction for switching the display item of the biological information displayed on the display unit 18 and the ten key 40 and setting button 42 which are used to feed attribute data relating to the living body such as the body height, age, and sex of the user are provided in the input unit 16. The switch button 26 is used to switch the items to be displayed after the measurement or to feed the attribute data. The display unit 18 displays the plural items of biological information measured by the measuring unit 6 or the attribute data fed by the ten key 40.

In Example 4, Table 1 indicating target values of plural items and Table 2 indicating the sex are prestored in the memory 12.

For example, three target values (three pieces of comparison object data) are stored in Table 1 of the memory 12 while correlated with the body height and sex as follows.

(Table 1)
1. 150,1,45,23,1050
2. 155,1,48,23,1120
3. 155,2,51,18,1225

(Table 2)
1. female
2. male

(Table 3)
1. weight value
2. body fat percentage
3. basal metabolism

(Table 4)
1. red
2. blue

Table 1 is a table which indicates the display mode data number, body height, sex, plural items of target values (weight value, body fat percentage, and basal metabolism) from the left. The display mode data on the first line of Table 1 is data indicating that the weight value (45 kg), subcutaneous fat ratio (23%), basal metabolism (1050 kcal) are sequentially displayed for a female (1) having a body height of 150 cm. Tables 3 and 4 are used in producing the display mode data.

The following description will be made with reference to Fig. 17. The user operates the ten key 40 of the input unit 16 to feed the personal attribute data (S51). When the user presses the setting button 42 of the input unit 16, the control unit 10 encourages the user to feed the attribute data in the order of the body height and the sex through the display unit 18. For example, the user feeds "151" with the ten key 40 when a message for encouraging the user to feed the "body height" is displayed on the display unit 18. Then, when the user presses the switch button 26, the control unit 10 stores the body height data of "151" in the memory 12, and the control unit 10 causes the display unit 18 to display the message for encouraging the user to feed "sex". The user feeds the sex data of "1" indicating the female using the ten key 40. The control unit 10 determines the comparison object data (first line) from the plural pieces of comparison object data (pieces of data of first to third lines) of Table 1 based on the fed attribute data (151,1) (S52). At this point, it is assumed that the target values of the plural items include the weight value of 45 kg, body fat percentage of 23%, and basal metabolism of 1050 kcal.

Then, the user performs the measurement with the biological information measuring apparatus 2. The measuring unit 6 detects the plural items of biological information using the sensor 20, and the computation unit 22 produces the measurement data relating to each item (S53 and S54). The computation unit 22 transfers the measurement data to the control unit 10. For example, the pieces of measurement data are the weight value of 55 kg, body fat percentage of 26%, and basal metabolism of 980 kcal.

The control unit 10 computes how many percent of a difference between the measurement value and the target value based on the target value. The control unit 10 performs the computation for each item to obtain comparison result H. For example, the control unit 10 obtains the comparison result using the following equation (1).

$$H=\alpha\times\{(\text{target value-measurement value})/\text{target value}\}\times100\} \ (\alpha=1 \text{ or } -1)$$

$$(1)$$

(Comparison Result H)

$$\text{weight value}=-\{(45-55)/45\}\times100\}\cong22.2 \ (\%)$$

$$\text{body fat percentage}=-\{(23-26)/23\}\times100\}\cong13.0 \ (\%)$$

$$\text{basal metabolism}=\{(1050-980)/1050\}\times100\cong6.6 \ (\%)$$

The control unit 10 performs the computation while setting the coefficients $\alpha$ of the weight and body fat percentage at "-1". The control unit 10 performs the computation while setting the coefficient $\alpha$ of the basal metabolism at "+1". The coefficient $\alpha$ is preset for each item. The coefficient of each item is stored in the memory 12.

Using Tables 3 and 4, the control unit 10 produces the pieces of display mode data in order that the comparison results are displayed in the descending order. For example, the control unit 10 produces the display mode data of "1,1,1,2,1,3,1" to store the display mode data of "1,1,1,2,1,3,1" in the memory 12. The display mode data indicates the "the number of display items (1), weight value (1), red (1), body fat percentage (2), red (1), basal metabolism (3), and red (1)". The control unit 10 causes the display unit 18 to display the measurement data one by one in the order of the weight value, body fat percentage, and basal metabolism. The control unit 10 performs control such that a background color of the display unit 18 is displayed in red when each item is displayed because each measurement result reaches the target value.

The control unit 10 rearranges the received pieces of measurement data according to the determined display mode data, and the control unit 10 causes the display unit 18 to display "weight value" (S55). When the control unit 10 detects that the switch button 26 is pressed (S56), the control unit 10 causes the display unit 18 to display "body fat percentage" (S57 and S55). Then, when the control unit 10 detects that the switch button 26 is pressed (S56), the control unit 10 causes the display unit 18 to display "basal metabolism" (S57 and S55). The display unit 18 turns off the power supply 14 when a predetermined time has elapsed since the display start (S58). The display mode of the measurement result is not limited to the method of displaying the measurement result one by one, but the method of displaying at least two measurement results may be listed.

In Example 4, the measurement result can be displayed on the display unit 18 in the display mode based on the measurement result comparison of the biological information on the user.

(Modification)

The target value is not limited to the ideal target value, but the measurement data measured in the past time may be used as the target value. The target value may be a statistical average value obtained from the attribute data relating to a living body. In Example 4, the personal attribute data is fed to determine the display mode. Alternatively, the user may feed the target value for each item to determine the display mode.

(Other Modifications)

In the above-described Examples, the plural items of biological information are measured by the body composition meter. However, the invention can also be applied to a pedometer and a blood-pressure meter.

In the pedometer, the number of steps, the number of steps in which the user walks with predetermined intensity or more, a time during which the user walks with predetermined intensity or more, consumed calories, a fat-burning amount, and a walking distance can be used as the plural items of biological information.

In the blood-pressure meter, a maximum blood pressure in the morning, a minimum blood pressure in the morning, a maximum blood pressure in the night time, and a minimum blood pressure in the night time can be used as the plural items of biological information.

**Claims**

1. A biological information measuring apparatus comprising:

   a storage unit in which display mode data is stored, said display mode data being used to determine a display mode of a plurality of items of biological information;
   a measuring unit which measures said plurality of items of biological information;
   a display unit which determines said display mode of said biological information measured by said measuring unit; and
   a control unit which produces said display mode data to control said display mode of said display unit according to said display mode data, said display mode data including data indicating display order of said plurality of items of biological information based on input data.

2. A biological information measuring apparatus comprising:

   an input unit which feeds display mode data used to determine a display mode of a plurality of items of biological information;
   a storage unit in which said display mode data is stored;
   a measuring unit which measures said plurality of items of biological information;
   a display unit which determines said display mode of said biological information measured by said measuring unit; and
   a control unit which causes said display unit to display said measured biological information according to said display mode stored in said storage unit.

3. The biological information measuring apparatus according to claim 2, wherein said input unit includes a switch button and a determination button, said switch button switching an item of said biological information displayed on said display unit, said determination button determining display order of an item of said displayed biological information.

4. The biological information measuring apparatus according to claim 2, wherein said input unit includes a plurality of item buttons relating to said biological information to be displayed on said display unit.

5. A biological information measuring apparatus comprising:

   a storage unit in which display mode data is stored, said display mode data being used to determine a display mode of a plurality of items of biological information;
   a measuring unit which measures said plurality of items of biological information;
   a display unit which determines said display mode of said measured biological information;
   an input unit which provides an instruction for switching display of an item of said biological information displayed on said display unit; and
   a control unit which produces display-related data relating to said display of said biological information displayed on said display unit in response to said instruction from said input unit, stores display mode data based on said display-related data in said storage unit, and controls said display mode of said display unit according to said stored display mode data.

6. The biological information measuring apparatus according to claim 5, wherein said display-related data is data indicating a time during which said biological information is displayed on said display unit.

7. The biological information measuring apparatus according to claim 5, wherein said display-related data is data indicating the number of times in which said instruction for switching said display is provided for each item of said biological information.

8. A biological information measuring apparatus comprising:

an input unit which feeds attribute data relating to a living body;

a storage unit in which a plurality of pieces of display mode data are prestored, said plurality of pieces of display mode data being used to determine a display mode of a plurality of items of biological information;

a measuring unit which measures said plurality of items of biological information;

a display unit which determines said display mode of said biological information measured by said measuring unit; and

a control unit which determines one piece of display mode data from a plurality of pieces of display mode data based on said attribute data and causes said display unit to display said measured biological information according to said determined display mode data.

9. The biological information measuring apparatus according to claim 8, wherein said attribute data relating to said living body is data relating to a body height, an age, or a sex.

10. The biological information measuring apparatus according to claim 9, wherein said display mode data is a data indicating display order of said plurality of items of biological information which are correlated with said body height, said age, or said sex.

11. A biological information measuring apparatus comprising:

a storage unit in which a plurality of items of target values are stored;

a measuring unit which measures a plurality of items of biological information;

a display unit which determines a display mode of said biological information measured by said measuring unit; and

a control unit which produces display mode data used to determine said display mode of said measured biological information according to result of comparison between said measured biological information and said target value for each item and causes said display unit to display said biological information according to said display mode data.

12. The biological information measuring apparatus as in any one of claims 2 to 11, wherein said display mode data is data relating to display order of said plurality of items of biological information, or a size or a color of characters determining said display mode of said plurality of items of biological information.

Fig. 1

Fig. 2

4 Operation unit

18 Display unit

16 Input unit

10 Control unit, 12 Memory, 14 Power supply

8 Cable

6 Measuring unit

20 Sensor

22 Computation unit

EP 1 997 424 A1

Fig. 3

EP 1 997 424 A1

Fig. 4

4 Operation unit
18 Display unit
16 Input unit
10 Control unit, 12 Memory, 14 Power supply
2 4 I / F
6 Measuring unit
2 4 I / F
20 Sensor
22 Computation unit

Fig. 5

Fig. 6

S 1

Display order changing mode

S 2

Display item

S 3    S 4

Switch button pressed?

YES    Switch items

NO

S 5

Determination button pressed?

NO

YES

S 6

Determine item

S 7

Display order changing mode ended?

NO

YES

S 8

Display determinate order

END

Fig. 7

Display unit

1. Body fat percentage
2. Weight
3. Basal metabolism

Input unit

Mode

Reset

Item A

Item B

Item C

EP 1 997 424 A1

Fig. 8

S 1 1

Display order changing mode

S 1 2

Item button pressed?

YES

S 1 3

Display item

S 1 4

Display order changing mod ended?

NO

YES

S 1 5

Display determinated order

END

Fig. 9

18         16

| Display unit | Input unit |
|---|---|
| | |

Switch

26

Fig. 10

```
        ( S T A R T )
              |
              |         S 2 1
              v
    [Perform measurement]
              |
              |         S 2 2
              v
    [Display result] <----------+
              |                 |
              |         S 2 3   |
              v                 |
    [Perform count] <-------+   |
              |             |   |
              |     S 2 4   |   |
              v             |   |
      / Switch button \  NO |   |
     <                 >----+   |
      \   pressed?    /         |
              |                 |
            YES       S 2 5     |
              v                 |
      [Store time,              |
       Switch display]          |
              |                 |
              |     S 2 6       |
              v                 |
      / All items \   NO        |
     <             >------------+
      \ displayed? /
              |
            YES       S 2 7
              v
    [Determine display
         mode]
              |
              v
         ( E N D )
```

Fig. 11

Display contents   a, b, c, d · · ·

```
    |   |        |   |   |        |
  ──┼───┼────────┼───┼───┼────────┼──────
    |   |   a    | b | c |   b    |  · · ·
    |   |        |   |   |        |
```

Fig. 12

18    16    36

| Display unit | Input unit |
| --- | --- |
|  | Item C |
|  | Item A    Item B |

32    34

Fig. 13

```
                    ┌─────────────┐
                    │   START     │
                    └──────┬──────┘
                           │              S 3 1
                    ┌──────▼──────────┐
                    │Perform measurement│
                    └──────┬──────────┘
                           │              S 3 2
                    ┌──────▼──────┐
          ┌────────►│Display result│◄──────────────┐
          │         └──────┬──────┘                │
          │                │              S 3 3     │
          │             ╱──▼──╲                     │
          │           ╱ Item button ╲      NO       │
          │          ◄   pressed?    ►──────────────┘
          │           ╲           ╱
          │             ╲──┬──╱
          │           YES  │              S 3 4
          │         ┌──────▼──────┐
          │         │Perform count │
          │         └──────┬──────┘
          │                │              S 3 5
          │         ┌──────▼──────┐
          │         │ Store time,  │
          │         │ Switch display│
          │         └──────┬──────┘
          │                │              S 3 6
          │             ╱──▼──╲
          │    NO     ╱Predetermined╲
          └──────────◄ time elapsed? ►
                      ╲           ╱
                        ╲──┬──╱
                      YES  │              S 3 7
                    ┌──────▼──────┐
                    │Determine display│
                    │    mode      │
                    └──────┬──────┘
                           │
                    ┌──────▼──────┐
                    │    END      │
                    └─────────────┘
```

Fig. 14

Fig. 15

START

S 4 1
Feed personal attribute data

S 4 2
Determine display mode

S 4 3
Perform measurement

S 4 4
Rearrange measurement result

S 4 5
Display result

S 4 8
Predetermined time elapsed?

NO

S 4 6
Switch button pressed?

NO

YES

S 4 7
Switch display

YES
END

EP 1 997 424 A1

Fig. 16

Fig. 17

```
        ┌─────────────────┐
        │     START       │
        └─────────────────┘
                 │          ∽ S 5 1
                 ▼
    ┌──────────────────────────────┐
    │  Feed personal attribute data │
    └──────────────────────────────┘
                 │          ∽ S 5 2
                 ▼
        ┌─────────────────────┐
        │ Determine target value │
        └─────────────────────┘
                 │          ∽ S 5 3
                 ▼
        ┌─────────────────────┐
        │ Perform measurement │
        └─────────────────────┘
                 │          ∽ S 5 4
                 ▼
    ┌──────────────────────────────┐
    │ Determine display mode from  │
    │  difference with target value │
    └──────────────────────────────┘
                 │          ∽ S 5 5
                 ▼
        ┌─────────────────┐◄───────────────────┐
        │  Display result │                    │
        └─────────────────┘                    │
                 │                              │
                 │              ┌────────────┐  │
                 │              │  Switch    │ ∽ S 5 7
                 │              │  display   │  │
                 │              └────────────┘  │
                 │                    ▲ YES      │ NO
                 ▼                    │          │
           S 5 8 ∽               ╱──────────╲
          ╱──────────╲   NO     ╱ Switch button ╲
         ╱ Predetermined ╲─────►╲   pressed?   ╱──┘
         ╲ time elapsed? ╱       ╲──────────╱
          ╲──────────╱              ∽ S 5 6
              │ YES
              ▼
        ┌─────────────────┐
        │      END        │
        └─────────────────┘
```

<div align="center">

**INTERNATIONAL SEARCH REPORT**

</div>

| | International application No. |
|---|---|
| | PCT/JP2007/052365 |

**A. CLASSIFICATION OF SUBJECT MATTER**
*A61B5/00*(2006.01)i, *A61B5/05*(2006.01)i

According to International Patent Classification (IPC) or to both national classification and IPC

**B. FIELDS SEARCHED**

Minimum documentation searched (classification system followed by classification symbols)
A61B5/00, A61B5/05

Documentation searched other than minimum documentation to the extent that such documents are included in the fields searched
Jitsuyo Shinan Koho          1922-1996     Jitsuyo Shinan Toroku Koho    1996-2007
Kokai Jitsuyo Shinan Koho    1971-2007     Toroku Jitsuyo Shinan Koho    1994-2007

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>A | JP 2005-237473 A (Tanita Corp.),<br>08 September, 2005 (08.09.05),<br>Full text; all drawings<br>& EP 1568318 A1          & 2005/187486 A1 | 1,2,4-6<br>3,7 |
| X<br>A | JP 6-78827 A (YAMAN Ltd.),<br>22 March, 1994 (22.03.94),<br>Full text; all drawings<br>& JP 2726197 B2 | 8,9<br>10 |
| X<br>Y | JP 2004-329412 A (Tanita Corp.),<br>25 November, 2004 (25.11.04),<br>Full text; all drawings<br>& EP 1472977 A1          & US 2004/220492 A1 | 11<br>12 |

| ☒ Further documents are listed in the continuation of Box C. | ☐ See patent family annex. |
|---|---|

* Special categories of cited documents:
"A" document defining the general state of the art which is not considered   to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search<br>    22 February, 2007 (22.02.07) | Date of mailing of the international search report<br>    06 March, 2007 (06.03.07) |
|---|---|
| Name and mailing address of the ISA/<br>    Japanese Patent Office | Authorized officer |
| Facsimile No. | Telephone No. |

Form PCT/ISA/210 (second sheet) (April 2005)

**INTERNATIONAL SEARCH REPORT**

| International application No. |
| --- |
| PCT/JP2007/052365 |

C (Continuation).   DOCUMENTS CONSIDERED TO BE RELEVANT

| Category* | Citation of document, with indication, where appropriate, of the relevant passages | Relevant to claim No. |
| --- | --- | --- |
| X<br>Y | JP 2003-288417 A  (Tanita Corp.),<br>10 October, 2003 (10.10.03),<br>Full text; all drawings<br>(Family: none) | 11<br>12 |
| Y | JP 2002-159450 A  (Misaki Inc.),<br>04 June, 2002 (04.06.02),<br>Full text; all drawings<br>(Family: none) | 12 |

Form PCT/ISA/210 (continuation of second sheet) (April 2005)

# EP 1 997 424 A1

| INTERNATIONAL SEARCH REPORT | International application No. |
|---|---|
| | PCT/JP2007/052365 |

**Box No. II**     **Observations where certain claims were found unsearchable (Continuation of item 2 of first sheet)**

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III**     **Observations where unity of invention is lacking (Continuation of item 3 of first sheet)**

This International Searching Authority found multiple inventions in this international application, as follows:

    The matter common to the inventions of claims 1-12 is "the biological
information measurement device having the measurement section for measuring
biological information, the display section for determining a display mode
of biological information, and the control section for controlling display
modes of the display section according to display mode data.
    However, the matter is not novel because the search has revealed that the
biological information measurement device having the above structure is
disclosed in JP 2005-237473 A (Tanita Corp.), 08 September 2005 (08.09.05),
full text, all the drawings.
    (continued to extra sheet)

1. ☐ As all required additional search fees were timely paid by the applicant, this international search report covers all searchable claims.

2. ☒ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**
the

☐ The additional search fees were accompanied by the applicant's protest and, where applicable, payment of a protest fee..

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2005)

**INTERNATIONAL SEARCH REPORT**

International application No.

PCT/JP2007/052365

Continuation of Box No.III of continuation of first sheet(2)

Since the above matter makes no contribution over the prior art, it is not a special technical feature within the meaning of PCT Rule 13.2, second sentence.

Accordingly, the inventions of claims 1-12 do not satisfy the requirement of unity of invention.

Form PCT/ISA/210 (extra sheet) (April 2005)

**REFERENCES CITED IN THE DESCRIPTION**

**Patent documents cited in the description**

- JP 2004243148 A

- JP 2005261488 A